# EUROPEAN PATENT APPLICATION

(11) **EP 0 894 502 A1**
(43) Date of publication of application: **03.02.1999**
(21) Application number: 97112205.6
(22) Date of filing: 30.07.1997
(51) Int. Cl.: A61L 15/18, A61L 15/20, A61L 15/46

(54) **Cyclodextrin-containing odour control material for absorbent articles**

(71) Applicant: THE PROCTER & GAMBLE COMPANY, Cincinnati, Ohio 45202 (US)
(72) Inventor: Carlucci, Giovanni, 66100 Chieti (IT); Guarracino, Mario, 64028 Silvi Marina, (Teramo) (IT); Di Cintio, Achille, 65126 Pescara (IT)
(74) Representative: Hirsch, Uwe Thomas

(57) **Abstract**

The present invention relates to an absorbent article having an odour control material incorporated therein for decreasing odours associated with bodily fluids. The odour control material is cyclodextrin having a particle size of greater than 12 µm. The odour control material offers the advantage of reduced cost compared to articles utilizing powdered cyclodextrin odour control material and thereby avoids the dust formation problems associated therewith.

## Description

This invention relates to an absorbent article comprising a odour control material and in particular to an article for absorbing fluids, for example, bodily fluids.

Absorbent articles are designed to be worn by humans to absorb bodily fluids, such as urine, menstrual fluid and perspiration, etc. Examples of absorbent articles include sanitary napkins, pantiliners, disposable diapers, incontinence pads, tampons and the like.

In use, the absorbent articles are known to acquire a variety of compounds, for example volatile fatty acids (e.g. isovaleric acid), ammonia, amines (e.g. triethylamine), fatty acids, sulphur containing compounds (e.g. mercaptans, sulphides), alcohols, ketones and aldehydes (e.g. furaldehyde) which release unpleasant odours. These compounds may be present in the bodily fluid or may be produced by fermentation once the bodily fluid is absorbed into the pad. In addition menstrual fluid which contains microorganisms can also generate malodorous by products. Unpleasant odours which emanate from absorbent pads when in use may make the wearer feel self conscious.

A number of compounds, mixtures, compositions and the like are known to combat some of the unpleasant odours referred to above many of which are based on absorbants such as activated carbon, clay and zeolites.

US-A-3939838 discloses an article for treating menstrual fluid having the function of effectively removing odour that is released from menstrual fluid. The odour controls exemplified are active carbon, active silica, active alumina, ion exchange resin and chlorophyll.

WO 89/02698 discloses absorbent compositions which are particularly useful as an improved animal or pet litter composition or as an absorbent for aqueous systems or oleophilic materials such as petroleum or vegetable oils. The composition comprises clay and a nitrogeneous compound absorber such as cyclodextrin.

Japanese Patent (A) 61-128973 describes a deodorizing agent comprising vegetable extract which contains at least one flavonoid, terpene or pyroligneous acid component and cyclodextrin or a derivative thereof. The deodorizing agent can be incorporated into a hygiene product such as a sanitary napkin or a disposable nappy. The invention disclosed in Japanese Patent (A) 61-128973 is based on the finding that cyclodextrin or a derivative thereof can be used as a solidifying agent to solve problems associated with solidifying known vegetable extracts without destroying their odour masking or deodorizing effects.

WO 94/22501 relates to odour removing compositions, suitable for use in absorbent articles such as catamenials and diapers, which comprise an effective amount of cyclodextrin having a particle size of less than 12µm. WO 94/22501 states that surface area availability of the uncomplexed cyclodextrin is essential for effective and efficient odour control performance by cyclodextrin powder. The small particles of the invention disclosed in WO 94/22501 must have a particle size of less than about 12µm, preferably less than about 10µm and most preferably less than about 5µm to provide a quick pick-up of odour when the cyclodextrin is wetted. The small particles can be prepared by grinding techniques eg using a fluid energy mill. Alternatively the small particle-size cyclodextrin can be obtained by means of an in situ and rapid crystallization where the substrate is impregnated with an aqueous solution of, e.g., uncomplexed β-cyclodextrin. Upon drying the cyclodextrin crystalizes out as small particles, adhering to the substrate and is immobilized on said substrate.

We have now surprisingly found that cyclodextrin acts as a very effective odour control material without the need to provide the cyclodextrin in small particle size. That is we have found that cyclodextrin having a particle size of greater than 12µm is a very effective odour control material.

The present invention thus provides a less expensive odour control material than that according to WO 94/22501 since the time-consuming and/or costly additional grinding steps or special crystallization techniques are not required.

Moreover, use of cyclodextrin having a larger particle size has the advantage that it allows easier incorporation of the odour control material in a product with less risk of dust formation. The latter is an important factor. Reduced dust means improved safety. Furthermore, the reduction of dust improves the quality of a laminate structure incorporating the odour control material. For example, in the formation of a laminate structure using a process according to WO 94/01069, any dust which is present may partially deactivate glue applied to the laminate side edges with an undesirable spilling of particles from the side edges. Use of cyclodextrin in larger particle size form avoids these problems.

Thus the present invention provides an absorbent article having incorporated therein an odour control material for decreasing odours associated with bodily fluids, characterized in that the odour control material is cyclodextrin having a particle size greater than 12µm.

The cyclodextrin preferably comprises a major portion with a particle diameter in the range of from 12µm to 800µm, most preferably with a mean particle size in the range of 20 to 350µm, most preferably in the range of from 30 to 160µm.

The particles of cyclodextrin may be agglomerated by a conventional procedure with, for example, a superabsorbent material. The agglomerated particle size range in this embodiment is 400 to 500µm, while the individual particle size is above 12µm.

According to a further aspect, the invention provides use of cyclodextrin having a particle size greater than 12µm as an odour-control material in an absorbent article.

It is known that cyclodextrins, such as β-cyclodextrin have a cage-like crystalline structure with a central cavity. The packing of the cyclodextrin molecules within the crystal lattice is such that the cavity of one cyclodextrin molecule is blocked off on both sides by adjacent cyclodextrins, thereby producing isolated and closed cavities. It has previously been disclosed (WO 94/22501) that surface area availability of the uncomplexed cyclodextrin is essential for effective and efficient odour control performance and consequently it was believed that particle sizes of less than about 12µm were required. We have surprisingly found that cyclodextrin have larger particle sizes ie greater than 12µm are effective odour-removing compounds.

Although the invention is not bound by theory, it is postulated that cyclodextrins having a particle size greater than 12µm are less soluble or dissolve more slowly and are therefore able to capture malodours over a larger period of time.

As used herein, the term "cyclodextrin" includes any of the known cyclodextrins such as unsubstituted cyclodextrins containing from six to twelve glucose units, especially, alpha-, beta;, and gamma-cyclodextrins, and/or their derivatives, and/or mixtures thereof. The alpha-cyclodextrin consists of 6, the beta-cyclodextrin 7, and the gamma-cyclodextrin 8, glucose units arranged in a donut-shaped ring. The specific coupling and conformation of the glucose units give the cyclodextrins a rigid, conical molecular structure with a hollow interior or a specific volume. The "lining" of the internal cavity is formed by hydrogen atoms and glycosidic bridging oxygen atoms, therefore this surface is fairly hydrophobic. These cavities can be filled with all or a portion of an organic molecule with suitable size to form an "inclusion complex". Alpha-, beta, and gamma-cyclodextrins can be obtained from, among others, American Maize-Products Company (Amaizo), Hammond, Indiana.

Alpha-, beta-, and gamma-cyclodextrin solids have "cage"-type crystalline structure. The packing of these cyclodextrin molecules within the crystal lattice is such that the cavity of one cyclodextrin molecule is blocked off on both sides by adjacent cyclodextrins, thereby producing isolated and closed cavities. These molecular arrangements are described in detail in "Cyclodextrin Technology, Jozsef Szejtlf, Kluwer Academic Publishers, 1988, page 6.

The individual cyclodextrins can also be linked together, e.g., using multifunctional agents to form oligomers, polymers, etc. Examples of such materials are available commercially from Amaizo and from Aldrich Chemical Company (beta-cyclodextrin/epichlorohydrin copolymers).

The preferred cyclodextrin is beta-cyclodextrin.

A commercially available β-cyclodextrin which is suitable for use in the present invention is Rhodocap-N Beta 88168 by Rhone-Poulenc which analyses as 10% particles having a particle size <7·7µm, 90% having a particle size less than 111·5 µm with a mean size of 37·5µm.

Another suitable β-cyclodextrin showed 10% particles having a dimension less than 8·8µm; 90% particles having a dimension less than 160·3µm; and a mean particle size of 58·7µm.

As used herein, the particle size refers to the largest dimension of the particle and to the ultimate (or primary) particles. The size of these primary particles can be directly determined with optical or scanning electron microscopes. The slides must be carefully prepared so that each contains a representative sample of the bulk cyclodextrin. The particle sizes can also be measured by any of the other well-known methods, e.g., wet sieving, sedimentation, light scattering, etc. A convenient instrument that can be used to determine the particle size distribution of the dry cyclodextrin directly (without having to make a liquid suspension or dispersion) is the Malvern Particle and Droplet Sizer, Model 2600C, sold by Malvern Instruments, Inc., Southborough, Massachusetts. Some caution should be observed in that some of the dry particles may remain agglomerated. The presence of agglomerates can be further determined by microscopic analysis. Some other suitable methods for particle size analysis are described in the article "Selecting a particle size analyzer: Factors to consider," by Michael Pohl, published in Powder and Bulk Engineering, Volume 4 (1990), pp. 26-29.

The absorbent article may be a disposable absorbent product such as a sanitary napkin or a pantiliner, or a disposable diaper, an incontinence pad, a tampon or the like. The disposable absorbent product may be breathable. According to one aspect of the invention the absorbent article is a pantiliner.

The weight of the odour control material which may be used in the absorbent article can be readily determined by the skilled person bearing in mind the size of the absorbent article in question. For example a suitable quantity of odour control material which may be used in a pantiliner is from 0·01g to 2·0 g, preferably the quantity is from 0·02 to 1·0g.

Optionally, other conventional compounds may be included in the article together with the odour control material, for example one or more of the following may be incorporated, activated carbon, silica, zeolite absorbants, charcoal, anti-microbial agents, ionic absorbents such as an absorbent gelling material (AGM), perfumes or known odour control materials such as inorganic metal salts, for example inorganic salts of Zn. The quantity of those compounds which may be added can be readily determined by those skilled in the art.

According to one embodiment the odour control material is cyclodextrin alone. According to another embodiment the odour control material consists of a combination of cyclodextrin with zeolite and silica.

According to a further embodiment the odour control material consists of a combination of cyclodextrin with zeolite, activated carbon and silica.

More preferably AGM is included in the article together with the odour control material of the present invention.

The odour control material may be incorporated into the article by methods disclosed in the art, for example layered on the core of the absorbent material or mixed within the fibers of the absorbent core. The odour control material is preferably incorporated between two layers of cellulose tissue, optionally the material may be bonded between two cellulose tissue layers with, for example, a hot melt adhesive or any suitable bonding system, as described in WO 94/01069.

The invention will now be illustrated with reference to the detailed description and examples taken in conjunction with the accompanying Figures.

Figure 1 shows a cross sectional view of a commercially available Always pantiliner.

Figure 2 shows a cross sectional view of a pantiliner having an absorbent core comprising three cellulose tissue layers, the odour control material being incorporated between the first and second tissue layers.

Figure 3 shows a cross sectional view of a pantiliner having an absorbent core comprising three cellulose tissue layers, the odour control material being incorporated between the second and third tissue layers.

(Always is a Registered Trade Mark)

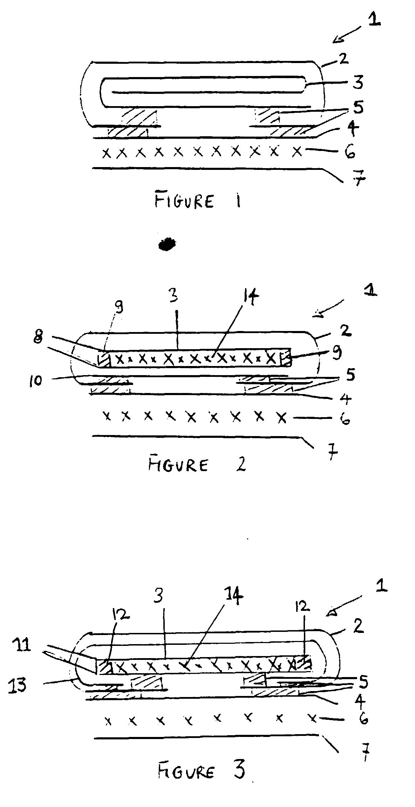

An absorbent article, namely a pantiliner which is an exemplary embodiment of an article into which the odour control material of the present invention may be incorporated, is shown in cross section in Figures 1 to 3.

The pantiliner may be of any shape known in the art, for example, rectangular, hour glass, winged, etc.

As shown in Figures 1 to 3 pantiliner 1 comprises a liquid pervious topsheet 2, an absorbent core 3, a liquid impervious backsheet 4, adhesive 5 which fastens the topsheet 2 to the backsheet 4 and the absorbent core 3 to topsheet 2, a layer of adhesive 6 which is secured to the backsheet 4 and which is covered by removable release liner 7. It is not, however, intended that the pantiliner should be limited to embodiments comprising all such elements. Additional elements known to the skilled person may also be included in the pantiliner.

Topsheet 2 is liquid permeable and, when pantiliner 1 is in use, is in close proximity to the skin of the user. Topsheet 2 is compliant, soft feeling and non-irritating to the user's skin. It can be made from any of the conventional materials for this type of use. Nonlimiting examples of suitable materials that can be utilized as the topsheet 2 are woven and nonwoven polyester, polypropylene, nylon and rayon and formed thermoplastic films, with formed films being preferred. Suitable formed films are described in US-A-4,324,246, US-A-4,324,214, US-A-4,341,217 and US-A-4,463,045.

The formed films are preferred for topsheet 2 because they are pervious to liquids and yet non-absorbent. Thus, the surface of the formed film, which is in contact with the body, remains dry and is more comfortable to the wearer. The topsheet may be constituted by the covering structure for sanitary products described in EP-A-0 207 904. Preferably the topsheet 2 is made of polyethylene perforated film (24.5g/m²)

The outer surface of topsheet 2 may be treated with a surfactant. Treating the outer surface of topsheet 2 with surfactant renders such surface more hydrophilic which results in liquid penetrating topsheet 2 faster than if the surface were not treated. It is preferred that the surfactant be substantially evenly and completely distributed throughout the outer surface of topsheet 2. This can be accomplished by any of the common techniques well known to those skilled in the art. For example, the surfactant can be applied to topsheet 2 by spraying, by padding or by the use of transfer rolls.

The inner surface of topsheet 2 may be secured in contacting relation to absorbent core 3. This contacting relationship results in liquid penetrating the topsheet 2 faster than if it were not in contact with absorbent core 3. Topsheet 2 can be maintained in contact with the absorbent core 3 by applying adhesive, preferably in spaced limited areas, to the inner surface of the topsheet 2. Examples of suitable adhesives used for such purpose include the acrylic emulsion E-1833BT manufactured by the Rohm & Haas Company, Philadelphia, Pennsylvania and the acrylic emulsion WB 3805 manufactured by H.B. Fuller Company of St. Paul, Minnesota. The adhesives can be applied by the same methods as the surfactant is applied to the outer surface of the topsheet 2.

Preferably the topsheet 2 wraps around the core 3, as shown in Figures 1 to 3, and is fastened by means of an adhesive 5 to backsheet 4.

Referring again to Figures 1 to 3, it can be seen that absorbent core 3 is positioned between topsheet 1 and backsheet 4. Absorbent core 3 provides the absorptive means for absorbing the bodily fluid. Absorbent core 3 is generally compressible, conformable and non-irritating to the user's skin. It can comprise any material used in the art for such purpose. Examples of such materials include multiple plies of creped cellulose wadding, fluffed cellulose fibers, wood pulp fibers also known as airfelt, textile fibers, a blend of fibers, a mass or batt of fibers, a web of polymeric fibers, a blend of polyester and polypropylene fibers, layers of cellulose tissue or layers of air laid tissue.

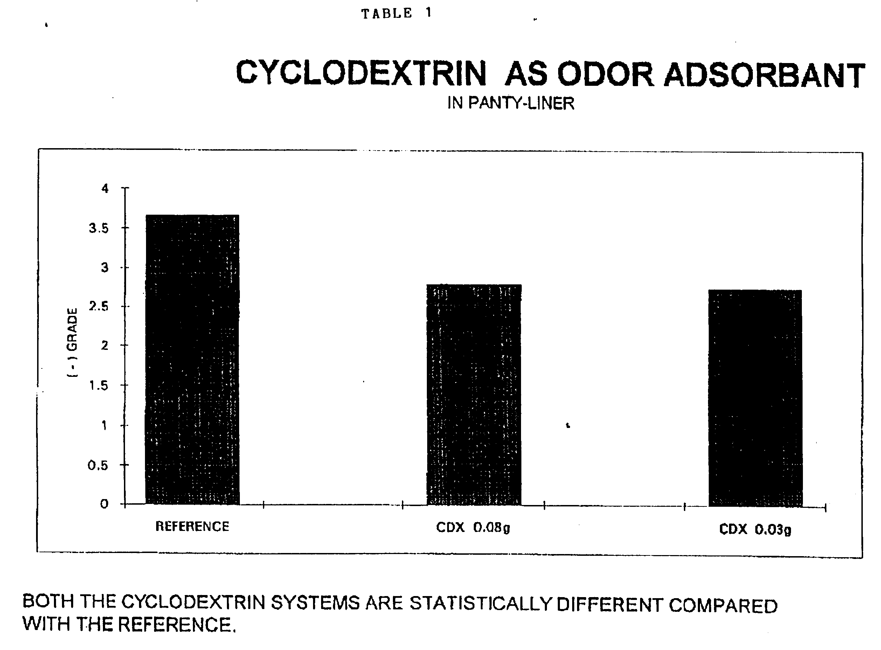

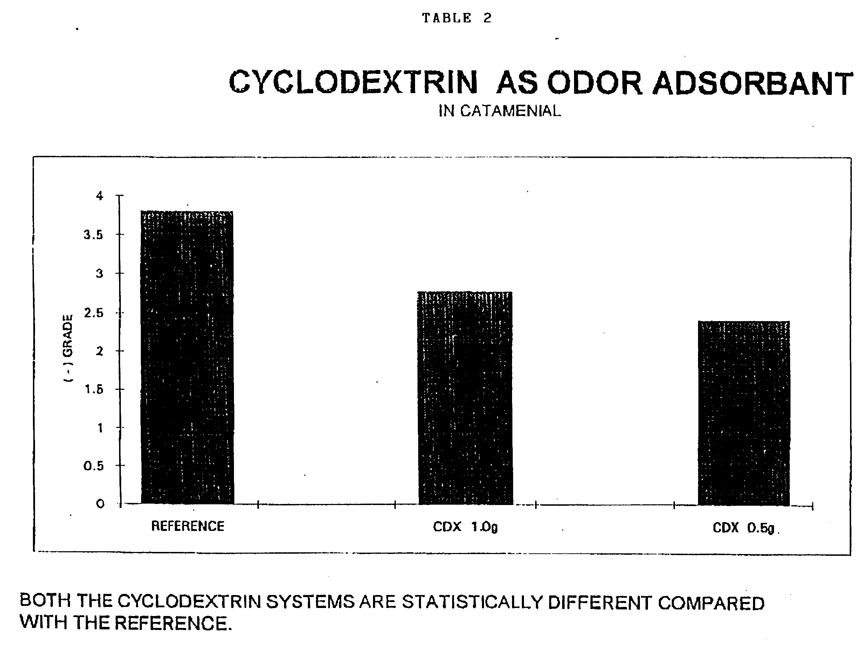

Preferably, the core comprises a mass or batt of fibers. A suitable core is the laminate described in WO 94/01069. While many types of fibers may be used, a preferred material is a batt of fluffed cellulose fibers. More preferably the core comprises cellulose tissue (40.61 g/m²) which forms three absorbent layers. Figure 1 shows an absorbent core 3 formed by one layer of cellulose tissue which has been folded as shown to form three absorbent layers. Figure 2 shows an absorbent core comprised of two layers of air laid cellulose tissue 8 joined at their longitudinal edges with adhesive 9 and having a layer of cellulose tissue 10 therebeneath to form the three layered absorbent core. Figure 3 shows two layers of air laid tissue 11 joined at their longitudinal edges with adhesive 12 and having a layer of cellulose tissue 13 wrapped therearound to form the third layer of the absorbent core.

Preferably, the odour control material disclosed herein is incorporated into the absorbent core by known techniques. It may, for example, be layered on the absorbent core or mixed with the fibers of the core. More preferably the odour control material 14 is placed between two layers of air laid cellulose tissue as shown in Figures 2 and 3 above.

Referring to Figures 1 to 3, the pantiliner is provided with a backsheet 4 which backsheet is impervious to liquids and, thus, prevents menstrual fluid which may be expressed from absorbent core 3 for soiling the body or clothing of the user. Suitable materials are well known in the art, including woven and nonwoven fabrics which have been treated to render them liquid repellent. Breathable or vapor pervious, liquid resistant materials, and those materials described in US-A-3,881,489 and US-A-3,989,86 can also be used. Preferred materials are those materials that provide additional fluid strikethough protection. Especially preferred materials include formed thermoplastic films. One especially suitable material is a polyethylene film having a thickness of from about .075 mils to about 1.25 mils, with a 1.0 mil thickness polyethylene film being especially suitable. Preferably the backsheet 4 is polyethylene embossed film (24.4 g/m²)

The outer surface of backsheet 4 is coated with adhesive 6. Adhesive 6 provides a means for securing the pantiliner in the crotch portion of a panty. Any adhesive or glue used in the art for such purpose can be used herein, with pressure sensitive adhesives being preferred. Suitable adhesives are Century A-305-IV manufactured by the Century Adhesives Corporation and Instant Lok 34-2823 manufactured by the National Starch and Chemical Corporation. Also, before pantiliner 1 is placed in use, the pressure sensitive adhesive 6 should be covered with removable release liner 7 in order to keep adhesive 6 from drying out or sticking to a surface other than the crotch portion of the panty prior to use. Any commercially available release liners commonly used for such purposes can be utilized herein. Nonlimiting examples of suitable release liners are BL 30 MG-A Silox E1/O and BL 30 MG-A Silox 4 P/O both of which are manufactured by the Akrosil Corporation. Preferably the release liner is a silicon paper having a thickness of about 45 µm (43.5g/m²). Other means which are known in the art may be used to affix the pantiliner in the crotch portion of a panty.

The invention will now be illustrated with reference to the examples wherein the article for absorbing bodily fluids is a pantiliner or a sanitary napkin. It will, of course, be appreciated that other absorbent articles may also have the odour control material incorporated therein, the incorporation of the odour control material into the pantiliner may be achieved by other known methods and the odour control material may be any of those disclosed in the present specification.

### Examples

### Example 1

### Incorporation of the odour control material into a pantiliner

The pantiliners used in the following examples were Always pantiliners (Always is a Registered Trade Mark) as sold by the Procter & Gamble Company. Each pantiliner was opened by cutting the polyethylene (PE) perforated film along a longitudinal edge of the product at its bottom face. The upper two layers of the inner three-folded cellulose tissue sheet, which constitutes the absorbent core of the product, were cut away and substituted with two layers of cellulose air laid tissue. The odour control material was homogeneously dispersed between said two layers. The whole pantiliner structure was then reconstituted and sealed along the edges by means of adhesive. Figure 2 represents a sectional view of the pantiliner structure which comprises a topsheet 2, airlaid layers 8 joined at their longitudinal edges with adhesive lines 9, the odour control material 14, a tissue layer 10, a backsheet 4, an adhesive layer 6 and a removable release liner 7.

Samples were prepared by the method as described above, which samples incorporate different cyclodextrins in two different amounts that is 0·09g and 0·03g.

The odour removing material was β-cyclodextrin with 10% particles having a dimension less than 8·8µm; 90% particles having a dimension less than 160·3µm; and a mean particle size of 58.7µm.

Particle size is determined using a Malvern API AeroSizer.

Using this apparatus, the particulate sample is first dispersed in air using either the dry powder feeder or, for suspensions, the specially designed nebulizer. The air-particle suspension is then passed through a nozzle and allowed to expand in a partial vacuum.

The air leaves the nozzles at near-sonic velocity and small particles are accelerated rapidly with the flow. Larger particles, due to their greater inertia, are accelerated more slowly. At a point downstream from the nozzle, the velocities of the individual particles are measured to determine their sizes.

In the measurement zone, the particles pass through two laser beams. As they do so, the light which they scatter is detected by two photomultipliers, one for each beam. The signals from the two detectors are analysed using correlation techniques to give each particle's time of flight between the two beams. This is achieved with an accuracy of better than one per cent. The analysis of the particle size showed:

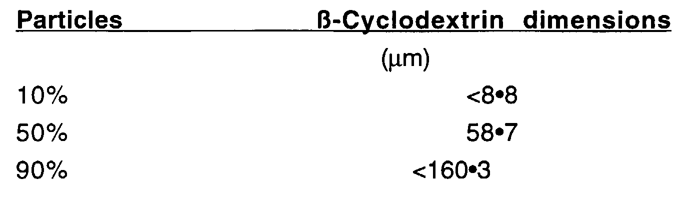

The odour removing material was assessed by the sniff test as subsequently described using pantiliners without the odour removing material as a reference. The results are shown in Table 1.

### Example 2

### Incorporation of the odour control material into a catamenial.

β-cyclodextrin was homogeneously incorporated in a batt of fluffed cellulose fibers.

The odour removing material was β-cyclodextrin commercially available as Rhodocap-N Beta 88168-10 by Rhone-Poulenc. The particle size was analysed as follows:

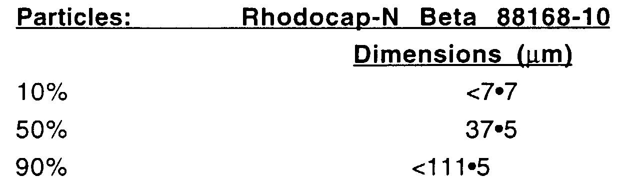

More than 55% of Rhodocap particles has a particle size diameter more than 45µm.

The odour removing material was assessed by the sniff test as subsequently described, in two different amounts viz 0·5g and 1·0g, using a catamenial without odour removing material as a reference.

The results are shown in Table 2.

### Odour Control Test Protocol

Each test comprises four separate stages which may be summarised as follows:
a) Consignment of the products.
b) Product return and preparation of the test samples.
c) Sniff-test.
d) Statistical analysis of the Data.

Each stage is described in more detail below.
a) Women were chosen who were known to have an odour control problem. Each of five women selected were given one product per test sample individually packaged in an anonymous bag. Every product was worn for seven hours.
b) The used product was placed into an aluminum tray, approximately 1 cm deep, covered with a perforated aluminum sheet, in order to keep it out of view, and finally covered with another tray of the same type, which was kept thereon in inverted position up to the moment of the sniff-test.
c) The sniff-test was performed in a "pre-ventilated" room by five graders. Each grader had been preselected for their sensitivity to the unpleasant smells present in an absorbent article after use and their ability to grade the unpleasantness of the odour in a consistent manner. Every grader evaluated the odour of each series of five products representing each sample using a pleasantness scale which ranges from -10 (highest level of unpleasantness) to 5 (most pleasant), passing though 0 (neutral rating). The pleasantness values for each sample were obtained as a mean of 25 observations (five graders, five products for each sample).
d) The results collected from the test were then analysed by statistical analysis software (SAS). The data was processed in order to show statistically significant differences between the treated and untreated products.

This difference is shown in the tables by means of a letter (in the "Significant difference" column) near every mean value; results with the same letter are not statistically significantly different.

The student's two-tailed "t" test was used to compare the data between two types of samples (see example 1), while Duncan's Multiple Range test was used to perform multiple comparisons (see Examples 2, 3 and 4).

Values of p<0.05 were considered statistically significant.

## Claims

1. An absorbent article having incorporated therein an odour control material for decreasing odours associated with bodily fluids, characterized in that the odour control material is cyclodextrin having a particle size greater than 12µm.

2. An absorbent article according to claim 1, wherein a major proportion of the cyclodextrin has a particle size in the range of from 12µm to 800µm.

3. An absorbent article according to claim 2, wherein the cyclodextrin has a mean particle size in the range of 20 to 350µm.

4. An absorbent article according to claim 3, wherein the cyclodextrin has a mean particle size in the range of 30 to 160µm.

5. An absorbent article according to claim 1, wherein the cyclodextrin is agglomerated with the agglomerated particles having a particle size of from 400 to 500 µm.

6. An absorbent article according to any of claims 1 to 5, wherein the cyclodextrin is β-cyclodextrin.

7. An absorbent article according to any of claim 1 to 6, wherein the absorbent article comprises a fluid-absorbing material selected from fibrous absorbent material, absorbent gelling material, absorbent foam, absorbent sponge and mixtures thereof.

8. An absorbent article according to any of claims 1 to 7, wherein the odour control material consists of the cyclodextrin alone.

9. An absorbent article according to any of claims 1 to 7, wherein the odour control material consists of a combination of the cyclodextrin with zeolite, activated carbon and silica.

10. An absorbent article according to any of claims 1 to 7, wherein the odour-control material consists of a combination of the cyclodextrin with zeolite and silica.

11. An absorbent article according to any of claims 1 to 10 which comprises AGM.

12. An absorbent article according to any of claims 1 to 11 which is a pantiliner or a sanitary napkin.

13. Use as an odour control material in an absorbent article of cyclodextrin having a particle size of greater than 12µm.
